(19) Europäisches Patentamt
European Patent Office
Office européen des brevets

(11) Veröffentlichungsnummer: **0 044 352**
**B1**

(12) **EUROPÄISCHE PATENTSCHRIFT**

(45) Veröffentlichungstag der Patentschrift: **20.02.85**

(51) Int. Cl.$^4$: **C 07 C 125/073,** A 61 K 6/08

(21) Anmeldenummer: **80104311.8**

(22) Anmeldetag: **23.07.80**

# (54) Addukte aus Diisocyanaten und Methacryloylalkylethern, -alkoxybenzolen bzw. -alkoxycycloalkanen und deren Verwendung.

(43) Veröffentlichungstag der Anmeldung:
**27.01.82 Patentblatt 82/04**

(45) Bekanntmachung des Hinweises auf die Patenterteilung:
**20.02.85 Patentblatt 85/08**

(84) Benannte Vertragsstaaten:
**AT BE CH DE FR GB IT LI LU NL SE**

(56) Entgegenhaltungen:
**DE-A-2 419 887**
**DE-B-2 357 324**
**US-A-3 425 988**

(73) Patentinhaber: **Blendax-Werke R. Schneider GmbH & Co.**
**Rheinallee 88**
**D-6500 Mainz (DE)**

(72) Erfinder: **Butler, David, V.**
**2825 E.Cortez Street**
**West Covina California 91791 (US)**
Erfinder: **Kidd, Patrick D.**
**1629 Yorkshire Court**
**San Dimas California 91773 (US)**
Erfinder: **Orlowski, Jan A.**
**1304 Rubio Street**
**Altadena California 91001 (US)**

**EP 0 044 352 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft neue, polymerisierbare Addukte aus Diisocyanaten und Methacryloylalkylethern, -alkoxybenzolen bzw. -alkoxycycloalkanen und deren Verwendung in Bindemitteln, insbesondere in Dentalmaterialien, sowie ein dentales Restorations- und Füllungsmaterial.

Es existieren bereits zahlreiche polymerisierbare Verbindung, die mehr als eine Doppelbindung im Molekül aufweisen. Diese Verbindungen werden für vielfältige Anwendungszwecke verwendet, insbesondere auch als Bindemittel zur Herstellung verschiedener Klebstoffe, u.a. auch in der Medizin und Dentalmedizin, bei der Konfektionierung von Knochenzementen, dentalen Restorations- und Füllmassen, dentalen Versiegelungsmaterialien, orthopädischen und orthodontischen Klebstoffen etc.

Es wurde nun gefunden, daß sich eine neue Klasse von Monomeren, die durch die Umsetzung von aliphatischen, aromatischen oder cycloaliphatischen Diisocyanaten mit Methacryloylalkylethern, -alkoxybenzolen oder -alkoxycycloalkanen erhalten werden, besonders gut für den Einsatz als Bindemittel und Klebstoffe, vor allem in den aufgezählten Arbeitsgebieten, eignet.

Gegenstand der Erfindung sind somit neue Verbindungen der allgemeinen Formel

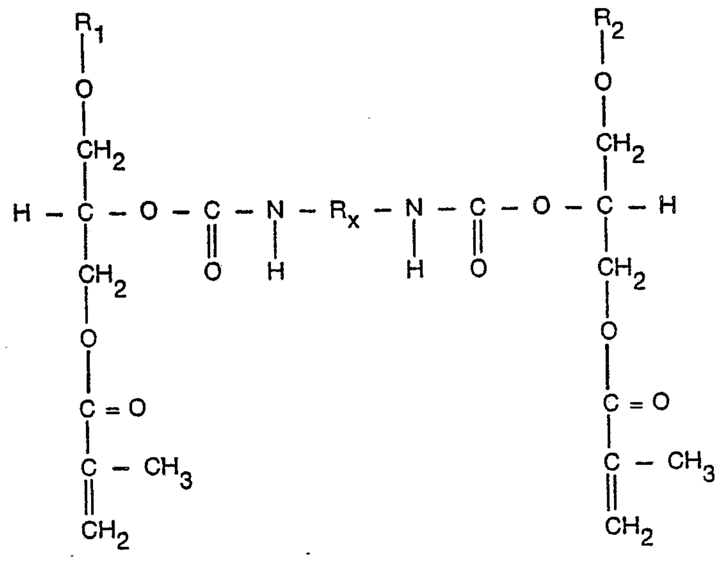

wobei $R_1$ und $R_2$ gleich oder verschieden sein können und eine geradkettige oder verzweigte Alkylgruppe mit 2 bis 14 C-Atomen oder eine gegebenenfalls substituierte Aryl- oder Cycloalkylgruppe und $R_x$ einen zweiwertigen (ar)aliphatischen, cycloaliphatischen oder aromatischen Rest mit 2 bis 14 C-Atomen bedeuten.

Wie gesagt, können die Reste $R_1$ und $R_2$ dabei gleich oder verschieden sein und Methyl-, Ethyl-, n-Propyl-, Isopropyl-, n- bzw. sec.- oder tert.-Butyl-, Pentyl-, Hexyl-, Octyl-, 2-Ethylhexyl-, Nonyl-, Decyl-, oder Laurylreste bedeuten, wobei sich gezeigt hat, daß mit dem 2-Ethylhexylrest besonders gute Ergebnisse erzielt werden können. $R_1$ und $R_2$ können jedoch auch gegebenenfalls substituiert aromatische Reste, insbesondere Phenyl-, Benzyl-, Toluyl- oder Xylyl-Gruppen oder Cycloalkyl-, insbesondere Cyclohexylreste, darstellen.

Der Rest $R_x$ steht für einen Alkylenrest, der verzweigt oder geradkettig sein kann, beispielsweise Ethylen-, Propylen-, Butylen-, Pentamethylen-, Hexamethylen-, Octamethylen-, oder 2-Ethylhexamethylenreste oder für zweiwertige aromatische Gruppen, insbesondere Phenylen-, Phenoxyphenylen-, Toluylen-, araliphatische Gruppen, wie Methylenbisphenyl- oder Propylenbisphenylreste sowie Cycloalkylengruppen, insbesondere Cyclohexan- bzw. Methylen- oder Propylenbiscyclohexyl-Gruppen.

Die Herstellung der erfindungsgemäßen polymerisierbaren Verbindungen mit 2 Methacrylgruppen erfolgt in der an sich bekannten Weise durch Kondensation der entsprechenden Isocyanate mit den entsprechenden Methacryloylalkylethern, -alkoxybenzolen bzw. -alkoxycycloalkanen, die ihrerseits wiederum durch Reaktion von Methacrylsäure mit den entsprechenden Glycidylethern in ebenfalls an sich bekannter Weise gewonnen wurden. Prinzipiell geeignete Herstellungsverfahren sind beispielsweise in der DE—AS 2 357 324 beschrieben.

Aus dieser DE—AS der DE—OS Nr. 2 419 887 sowie der US—PS Nr. 3 425 988 sind zwar ebenfalls bereits (Meth)Acrylsäureester bekannt, die Carbaminsäuregruppierungen im Molekül aufweisen, jedoch handelt es sich bei diesen bekannten Produkten um von den erfindungsgemäßen Monomeren strukturell unterschiedliche Produkte, mit denen die durch die Verwendung der erfindungsgemäßen Zusammensetzungen erzielbaren Vorteile nicht erreicht werden können.

Die erfindungsgemäßen Produkte eigenen sich grundsätzlich für alle diejenigen Verwendungszwecke, wo Monomere mit mindestens zwei polymerisierbaren Doppelbindungen im Molekül Einsatz finden können. Wie bereits angedeutet, sind sie besonders geeignet zur Anwendung als Bindemittel und in Klebstoffen, insbesondere zur Anwendung in der Medizin und Dentalmedizin.

In der Dentalmedizin haben besonders die aus Füllstoffen und polymerisierbaren Verbindungen bestehenden Füllungsmaterialien, die sogenannten "Composites", in den letzten Jahren zunehmende Bedeutung erlangt, da diese Produkte vom Zahnarzt leicht und sicher zu handhaben sind, vom Patienten in der Regel gut und reizlos vertragen werden und die Möglichkeit bieten, von den aus physiologischen Gründen immer wieder kritisierten Amalgam-Füllungsmaterialien abzurücken.

Nachteile der Composites gegenüber den herkömmlichen Füllungsmaterialien sind jedoch ihre Schrumpfungsanfälligkeit und ihre Wasserabsorption; besonders durch die mögliche Schrumpfung können zwischen Füllungsrand und ausgefüllter Kavität im Laufe der Zeit sekundärkariöse Erscheinungen auftreten.

Es ist daher das Bestreben der Fachwelt, Composites herzustellen, die nach Möglichkeit keine Schrumpfung aufweisen, ein geringe Wasserabsorption besitzen, gute mechanische Eigenschaften aufweisen und farbstabil sind.

Diese Eigenschaften lassen sich durch einen möglichst hohen Anteil des Füllermaterials in den Zusammensetzungen erreichen. Die Höchstmenge des einzusetzenden Fülleranteils ist jedoch abhängig von den Eigenschaften der in der Zusammensetzung eingesetzten Monomeren.

Übliche Composites weisen nach ihrer Aushärtung in der Regel folgende mechanische Eigenschaftswerte auf:

| | |
|---|---|
| Wasserabsorption bei 37°C: | 0,7—1,2 $mg/cm^2$ *) |
| Druckfestigkeit: | 30.000 bis 40.000 psi+) |
| Diametrale Zugfestigkeit: | 3.480 bis 4.200 psi*) ++) |
| Härte (Barcol): | 98 |
| Farbstabilität: | Farbveränderung nicht erkennbar*) |
| Opazität/Transluzenz-Faktor: | 0,35 bis 0,55*) |
| Gewichtsverhältnis Füller zu Harz: | 3,5:1 bis 4,2:1 |

*) bestimmt entsprechend der im "Journal of the American Dental Assoc., Vol. 94 (Juni 1977)" beschriebenen Specification No. 27.
+) $(=20685 \cdot 10^4 - 27580 \cdot 10^4$ Pa)
++) $(=23995 \cdot 10^3 - 28959 \cdot 10^3$ Pa)

Es wurde nun gefunden, daß man die aufgeführten mechanischen Eigenschaften üblicher, bekannter Composites durch den Einsatz der erfindungsgemäßen Monomeren ganz wesentlich verbessern kann und insbesondere ein wesentlich erhöhtes Verhältnis von Füllstoff zu Harz erreicht werden kann, was wiederum eine ebenso wesentliche Verbesserung der physikalischen Eigenschaften der Füllungen zur Folge hat.

Der Füllstoffanteil in der Gesamtzusammensetzung läßt sich dabei bis auf etwa 90% steigern.

Gegenstand der vorliegenden Erfindung ist daher auch ein dentales Füllungsmaterial, das gekennzeichnet ist durch einen Anteil zumindest eines der erfindungsgemäßen Monomeren, eines Füllers, eines Polymerisationsinitiators oder Beschleunigers, sowie gegebenenfalls weiterer an sich bekannter Stoffe wie weiterer Monomerer, UV-Absorber, Stabilisatoren, Farbstoffe etc.

Bei den eingesetzten Füllungsmaterialien kann es sich um röntgendurchlässige oder röntgenundurchlässige Materialien handeln. Beispiele sind insbesondere die verschiedenen Siliciumdioxid-Modifikationen, wie Glas (pulverisiertes Glas), Borsilicatglas und andere Gläser wie Quarzit, Christobalit u.ä. für röntgendurchlässige Füllstoffe und Bariumaluminiumsilicat, Lithiumaluminiumsilicat oder Glaskeramik-Füllstoffe, die beispielsweise die Elemente Lanthan oder Zirkonium enthalten, für röntgenundurchlässige Füllstoffe. Geeignete röntgenundurchlässige Füllstoffe sind beispielsweise in den US—PSen Nr. 3 801 344, 3 808 170 und 9 975 203 und der DE—OS Nr. 2 347 591 beschrieben.

Zur Erhöhung der Haftfestigkeit können diese Füllstoffe auch in bekannter Weise silanisiert sein.

Die Teilchengrößen der eingesetzten Füllstoffe bewegen sich in der Regel zwischen etwa 0,01 und 100 Mikron; es ist in vielen Fällen möglich und auch sinnvoll, Kominationen aus Füllstoffen niederer und höherer Teilchengrößen einzusetzen, wobei der bevorzugte Teilchendurchmesser zwischen etwa 0,05 und etwa 50, vorzugsweise etwa 30 Mikron liegt.

Composites liegen in zwei Modifikationen vor, entweder als zweiphasige Präparate, von denen die eine Phase einen Polymerisationsinitiator, beispielsweise ein Peroxid, und die andere Phase einen

Beschleuniger für dieses Peroxid, beispielsweise ein organisches Amin, enthält, wobei das Zusammenbringen beider Phasen unmittelbar vor der Zahnfüllung erfolgt und die Polymerisation in der aufgebohrten, vorzugsweise mit einem Unterfütterungsmaterial versehenen, zu füllenden Kavität eintritt.

Es ist jedoch auch möglich, einphasige Präparate herzustellen, die unter Einwirkung von Licht, beispielsweise UV- oder Laser-licht, polymerisieren und dann selbstverständlich einen Photopolymerisationsinitiator und gegebenenfalls auch einen Beschleuniger dafür enthalten müssen.

Der Einsatz der erfindungsgemäßen Monomeren ist selbstverständlich sowohl in Zwei- als auch in Einphasenpräparaten möglich.

Für Einphasenpräparate kommt in der Regel die Polymerisationseinleitung durch Strahlung in Betracht. Entsprechende Photopolymerisationsinitiatoren sind bekannt, vorzugsweise handelt es sich dabei um Carbonylverbindungen, insbesondere Benzil und Benzilderivate, wie 4,4-Oxydibenzil oder andere Dicarbonylverbindungen, z. B. Diacetyl, 2,3-Pentandion, oder Metallcarbonyle, Chinone und deren Derivate. Der Anteil an Photopolymerisationsinitiator beträgt etwa 0,01 bis etwa 5 Gew.-% der Gesamtzusammensetzung.

Diese einphasigen Präparate enthalten vorzugsweise auch noch sogenannte Polymerisationsbeschleuniger. Dies sind Substanzen, die in Gegenwart von Polymerisationsinitiatoren die Polymerisations reaktion beschleunigen.

Bekannte Beschleuniger sind beispielsweise verschiedene Amine wie p-Toluidin, Dimethyl-p-toluidin, Trialkylamine, Polyamine wie N, N, N', N'-Tetraalkylalkylendiamine und Sulfimide, vorzugsweise in einer Menge von etwa 0,01 bis etwa 5 Gew.-% der Gesamtzusammensetzung.

Soll das die erfindungsgemäßen Monomeren enthaltende Dentalfüllungsmaterial nicht lichthärtbar sein und in zwei bis zur Anwendung voneinander getrennt gehaltenen Phasen vorliegen, so enthält eine dieser Mischungen in der Regel einen Polymerisationsinitiator. Dies sind zumeist Peroxide, die bei der Auslösung der Polymerisation unter Radikalbildung zerfallen. Geeignete Peroxide sind beispielsweise Arylperoxide wie Benzoylperoxid, Cumolhydroperoxid, Harnstoffperoxid, tert.-Butylhydroperoxid oder -perbenzoat und Silylperoxide, in Mengen von vorzugsweise etwa 0,01 bis etwa 5, insbesondere etwa 0,5 bis 2,5 Gew.-%.

Enthält die eine Phase des zweiphasigen Mittels einen Polymerisationsinitiator, so wird der anderen Phase zweckmäßigerweise ein Beschleuniger des oben beschriebenen Typs, vorzugsweise ein Amin, zugesetzt.

In diesen die erfindungsgemäßen Monomeren enthaltenden Dentalfüllungsmaterialien können zur Verbesserung der Haftfähigkeit zwischen Füllstoff und Harz polymerisierbare Organosiliciumverbindungen mitverwendet werden, beispielsweise Methacryloylalkyltrihydroxysilane oder Methacryloyltrimethoxysilan.

Zusätzlich zu den erfindungsgemäßen Monomeren können in den eingesetzten dentalen Füllungsmaterialien weiter für diesen Zweck bereits vorgeschlagene Monomere mit verwendet werden. Solche Monomere sind beispielsweise Alkandioldimethacrylate wie 1,6-Hexandioldimethacrylat, 1,4-Butandioldimethacrylat oder Tri- oder Tetraäthylenglykoldimethacrylat, Bis-(2-methacryloyläthyl)-phthalat, -isophthalat oder -terephthalat, Trimethylolpropandi- und trimethacrylat, Reaktionsprodukte aus Diisocyanaten und einfachen Hydroxyalkylmethacrylaten, wie sie beispielsweise in der DE—OS Nr. 2 312 559 beschrieben sind, Reaktionsprodukte aus Bisphenolen, insbesondere Bisphenol A, und Glycidylmethacrylat (Bis-GMA), Addukte aus (Di-)Isocyanaten und 2,2-Propanbis-[3-(4-phenoxy)-1,2-hydroxypropan-1-methacrylat] nach der US—PS Nr. 3 629 187 sowie sonstige für diesen Zweck bereits vorgeschlagene polymerisierbare Verbindungen.

Zur Einstellung eines möglichst naturgetreuen Eindrucks der gefüllten Zahnflächen enthalten Composite-Materialien zuweilen Farbstoffe in geringen Mengen.

Weiterhin ist der Zusatz geringer Mengen an UV-Stabilisatoren möglich. Geeignete Stabilisatoren sind z.B. Hydrochinon, p-Benzochinon, p-Butylhydroxytoluol u.ä.

Die folgenden Beispiele dienen der Erläuterung der Erfindung:

## Beispiel A

225 g Phenylglycidyläther wurden zusammen mit 150 g Methacrylsäure bei 100°C in Gegenwart von 1 g Triphenylphosphin und 0,7 g di-tert.-Butyl-p-hydroxytoluol unter Rühren umgesetzt, bis ein Epoxyäquivalent von weniger als 0,005 erreicht war.

Nach der Abdestillation des unreagierten Methacrylsäure-Überschusses bis zum Erreichen einer Säurezahl unterhalb 3 wurde der verbleibende Rückstand auf 40° gekühlt und die äquimolare Menge Methylen-bis-(4-isocynatocyclohexan) während einer Stunde bei 45—50°C zugesetzt. Die Mischung wurde bei 30°C über Nacht stehen gelassen.

Das erhaltene Carbamat ist eine hellgelbe Flüssigkeit mit einem Brechungsindex von 1,528 bei 30°C.

Auf analoge Weise kann durch Austausch des Phenylglycidylethers durch Cyclohexylglycidylether das entsprechende Cyclohexylderivat hergestellt werden.

## Beispiel B

Ein analog Beispiel A erhaltenes Reaktionsprodukt aus 150 g Phenylglycidylether und 129 g Methacrylsäure wurde bei 45—50°C während einer Stunde mit 84 g Hexamethylendiisocyanat versetzt.

Nach dem Stehenlassen über Nacht bei 30°C wurde eine blaßgelbe viskose Flüssigkeit, bestehend aus dem Hexamethylenbiscarbamat des 3-Methacroyl-2-hydroxypropoxybenzols, erhalten.

Beispiel C

185,34 g 2-Ethylhexylglydidylether und 129 g Methacrylsäure wurden bei 100°C in Gegenwart von 2 g Triphenylphosphin unter starkem Rühren umgesetzt, bis das Reaktionsgemisch ein Epoxyäquivalent von weniger als 0,005 aufwies.

Nach dem Abdestillieren des Methacrylsäureüberschusses bis zum Erreichen einer Säurezahl von weniger als 3 wurde dis Mischung auf 40°C gekühlt und während einer Zeitspanne von 1 Stunde 131 g Methylenbis-(4-isocyanatocyclohexan) zugesetzt bei 45—50°C. Danach wurde das Reaktionsprodukt über Nacht bei 30°C gelagert.

Das Reaktionsprodukt ist eine bleichgelbe viskose Flüssigkeit mit einem Brechungsindex von 1,543 bei 30°C.

Beispiel 1

Ein "Composite"-Füllungsmaterial wurde aus folgenden Bestandteilen hergestellt:

| Paste A | | Paste B | |
|---|---|---|---|
| Pulverisiertes Quarz | 200 Teile | Pulverisiertes Quarz | 200 Teile |
| Pulverisiertes Glas | 320 Teile | Pulverisiertes Glas | 320 Teile |
| Methacroylpropyltri-hydroxysilan | 4 Teile | Methacroylpropyltri-hydroxysilan | 4 Teile |
| Benzoylperoxid | 2,5 Teile | N,N-Diethanol-p-toluidin | 4 Teile |
| Bis-GMA | 72 Teile | UV-Absorber (Cyasorb$^R$) | 1 Teil |
| 2,2-Bis-[4'-(2"-methacroyl-ethoxy-)phenyl-]propan | 66 Teile | Monomeres entsprechend Beispiel C | 70 Teile |
| Triethylenglykol-dimethacrylat | 22 Teile | Hexandioldimethacrylat | 30 Teile |

Die Pasten A und B wurden zu gleichen Teilen zusammengemischt und kurzzeitig ausgehärtet; das erhaltene Produkt zeigte folgende mechanische Eigenschaften:

| | |
|---|---|
| Wasserabsorption bei 37°C: | 0,51 mg/cm$^2$ |
| Diametrale Zugfestigkeit: | 6100 psi (42060 · 10$^3$ Pa) |
| Druckfestigkeit: | 38000 psi (26201 · 10$^4$ Pa) |
| Härte (Barcol): | 100 |
| Opazität/Transluzenz (Faktor $C_{70}$): | 0,4 |

Das Material zeigte darüberhinaus eine ausgezeichnete Farbstabilität.

Beispiel 2

Ein "Composite"-Füllmaterial bestand aus 2 Pasten folgender Zusammensetzung:

| Paste A | | Paste B | |
|---|---|---|---|
| Pulverisiertes Glas | 500 Teile | Pulverisiertes Glas | 500 Teile |
| Methacroylpropyltri-hydroxysilan | 4 Teile | Methacroylpropyltri-hydroxysilan | 4 Teile |
| Benzoylperoxid | 2,5 Teile | N,N'-Diethanol-p-toluidin | 4 Teile |

| | | | |
|---|---|---|---|
| Monomeres entsprechend Beispiel B | 75 Teile | UV-Absorber (Cyasorb®) | 1 Teil |
| Hexandioldimethacylat | 25 Teile | Monomeres entsprechend Beispiel B | 75 Teile |
| | | Hexandioldimethacrylat | 25 Teile |

Die Pasten A und B wurden in etwa gleichen Mengen miteinander vermischt und waren nach etwa 2,5 Minuten bei 23°C vollständig ausgehärtet. Das Polymerisationsprodukt zeigte die folgenden physikalischen Werte:

| | |
|---|---|
| Wasserabsorption bei 37°C: | 0,45 mg/cm$^2$ |
| Diametrale Zugfestigkeit: | 6200 psi (42749 · $10^3$ Pa) |
| Druckfestigkeit: | 40000 psi (2758 · $10^5$ Pa) |
| Härte (Barcol): | 100 |

Das Produkt zeigte hervorragende Farbstabilität und ein ansprechendes, natürliches Aussehen.

Beispiel 3

Ein aus 2 Pasten bestehendes Füllungsmaterial wurde hergestellt:

| Paste A | | Paste B | |
|---|---|---|---|
| Pulverisiertes Glas | 500 Teile | Silica | 500 Teile |
| Methacroylpropyltrihydroxysilan | 4 Teile | Methacroylpropyltrihydroxysilan | 4 Teile |
| Benzoylperoxid | 2,5 Teile | N,N-Diethanol-p-toluidin | 4 Teile |
| Monomeres entsprechend Beispiel A | 70 Teile | UV-Absorber | 1 Teil |
| Triethylenglykoldimethacrylat | 30 Teile | Monomeres entsprechend Beispiel A | 70 Teile |
| | | Hexandioldimethacrylat | 30 Teile |

Die Pasten A und B wurden zu etwa gleichen Teilen gemischt. Die Aushärtung erfolgte bei 23°C in 150 Sekunden. Das ausgehärtete Material zeigte folgende Eigenschaften:

| | |
|---|---|
| Wasserabsorption bei 37°C: | 0,45 mg/cm$^2$ |
| Diametrale Zugfestigkeit: | 6500 psi (44818 · $10^3$ Pa) |
| Druckfestigkeit: | 40500 psi (279248 · $10^3$ Pa) |
| Härte (Barcol): | 100 |

Die Farbstabilität war ausgezeichnet.

Beispiel 4

Es wurde ein radioopakes, lichthärtendes Einphasen-Füllungsmaterial der folgenden Zusammensetzung hergestellt:

| | |
|---|---|
| Titansilicat (mittl. Teilchengröße 0,1—5 Mikron) | 190 Teile |
| Lithiumaluminiumsilikat (mittl. Teilchengröße 5—25 Mikron) | 700 Teile |

| | |
|---|---|
| Acetophenon | 3 Teile |
| N,N-Dimethyl-p-toluidin | 5 Teile |
| Addukt aus Methacryloylpropoxyhydroxycyclohexan und Toluylendiisocyanat | 80 Teile |
| Bis-GMA | ·26 Teile |

Beispiel 5

Ein Fissurenversiegelungsmaterial weist folgende Monomeren-Zusammensetzung auf:

| | |
|---|---|
| Monomeres entsprechend Beispiel A | 50% |
| Triethylenglykoldimethacrylat | 20% |
| 1,6-Hexandioldimethacrylat | 30% |

Die Härtung dieses Harzgemisches kann entweder durch ein Redoxsystem (z.B. Cumolperoxid/Triarylamin) oder durch Bestrahlung mit UV-Licht oder sichtbarem Licht in Gegenwart eines UV-Aktivators wie Benzoin, Benzoinmethylether, Benzil, u.ä. erfolgen.

Beispiel 6

Dentallack:

| | |
|---|---|
| Addukt aus Hexamethylendiisocyanat und 3-Methacroyloxy-2-hydroxypropyoxytoluol | 75% |
| 2-Ethylhexylmethacrylat | 10% |
| Glykoldimethacrylat | 14,5% |
| Benzoinmethylether | 0,5% |

Dieser Lack ist UV-härtend und kann in der Dentalmedizin beliebig eingesetzt werden. Es sei nochmals betont, daß sich die erfindungsgemäßen Verbindungen zum Einsatz für alle Zwecke eignen, wo Monomere mit mehr als einer Doppelbindung im Molekül in Bindemitteln zum Einsatz gelangen, insbesondere in der Medizin und Dentalmedizin.

**Patentansprüche für die Vertragsstaaten: BE CH DE FR GB IT LI LU NL SE**

1. Verbindungen der allgemeinen Formel

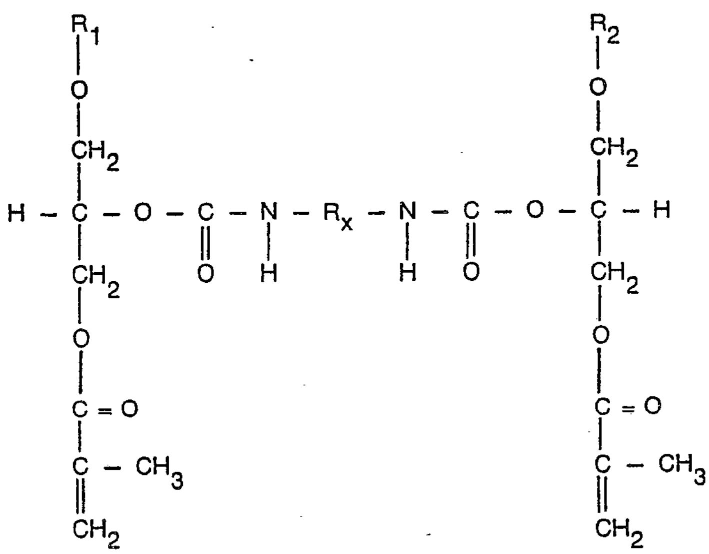

wobei $R_1$ und $R_2$ gleich oder verschieden sein können und eine geradkettige oder verzweigte Alkylgruppe mit 1 bis 14 C-Atomen, eine gegebenenfalls substituierte Phenyl- oder Cycloalkylgruppe, eine Benzylgruppe und $R_x$ einen zweiwertigen aliphatischen, cycloaliphatischen, araliphatischen oder aromatischen Rest mit 2 bis 14 C-Atomen bedeuten.

2. Verbindungen der allgemeinen Formel nach Anspruch 1, dadurch gekennzeichnet, daß $R_1$ und $R_2$ $C_2$—$C_8$-Alkylreste darstellen und $R_x$ einen Hexamethylenrest bedeutet.

3. Verbindungen der allgemeinen Formel nach Anspruch 1, dadurch gekennzeichnet, daß $R_1$ und $R_2$ $C_2$—$C_8$-Alkylreste darstellen und $R_x$ einen Phenylen- oder Toluylenrest bedeutet.

4. Verbindungen der allgemeinen Formel nach Anspruch 1, dadurch gekennzeichnet, daß $R_1$ und $R_2$ $C_2$—$C_8$-Alkylreste darstellen und $R_x$ einen Cycloalkylen- oder Methylenbiscycloalkylrest bedeutet.

5. Verbindungen nach einem der Ansprüche 2 bis 4, dadurch gekennzeichnet, daß $R_1$ und $R_2$ 2-Äthylhexylreste darstellen.

6. Verbindungen der allgemeinen Formel nach Anspruch 1, dadurch gekennzeichnet, daß $R_1$ und $R_2$ gegebenenfalls substituierte Phenylreste darstellen und $R_x$ einen Hexamethylenrest bedeutet.

7. Verbindungen der allgemeinen Formel nach Anspruch 1, dadurch gekennzeichnet, daß $R_1$ und $R_2$ gegebenenfalls substituierte Phenylreste und $R_x$ eine Phenylen- oder Toluylenrest bedeuten.

8. Verbindungen der allgemeinen Formel nach Anspruch 1, dadurch gekennzeichnet, daß $R_1$ und $R_2$ gegebenenfalls substituierte Phenylreste und $R_x$ einen Cycloalkylen- oder Methylenbiscycloalkylrest bedeuten.

9. Verbindungen der allgemeinen Formel nach Anspruch 1, dadurch gekennzeichnet, daß $R_1$ und $R_2$ gegebenenfalls substituierte Cycloalkyl-, insbesondere Cyclohexylreste, und $R_x$ einen Hexamethylenrest bedeuten.

10. Verbindungen der allgemeinen Formel nach Anspruch 1, dadurch gekennzeichnet, daß $R_1$ und $R_2$ gegebenenfalls substituierte Cycloalkyl-, insbesondere Cyclohexylreste, und $R_x$ einen Phenylen- oder Toluylenrest bedeuten.

11. Verbindung der allgemeinen Formel nach Anspruch 1, dadurch gekennzeichnet, daß $R_1$ und $R_2$ gegebenenfalls substituierte Cycloalkyl-, insbesondere Cyclohexylreste, und $R_x$ einen Cycloalkylen- oder Methylen-biscycloalkylrest bedeuten.

12. Verwendung einer oder mehrerer Verbindungen nach den Ansprüchen 1 bis 11 als Bindemittel.

13. Verwendung einer oder mehrerer Verbindungen nach den Ansprüchen 1 bis 11 als Bindemittel in dentalen Behandlungsmaterialien.

14. Dentales Restorations- und Füllungsmaterial, dadurch gekennzeichnet, daß es neben Füllstoffen, Initiatoren, Beschleunigern und weiteren üblichen Bestandteilen mindestens eine Verbindung nach den Ansprüchen 1 bis 11 enthält.

15. Dentales Restorations- und Füllungsmaterial nach Anspruch 14, dadurch gekennzeichnet, daß es die folgenden Bestandteile enthält:

9—90 Gew.-% mindestens einer Verbindung nach den Ansprüchen 1 bis 11;
0,01—2,5 Gew.-% mindestens eines Polymerisationsinitiators oder Polymerisationsbeschleunigers;
10—85 Gew.-% mindestens eines Füllstoffs;

sowie gegebenenfalls weitere polymerisierbare Verbindungen, UV-Absorber, Farbstoffe, Haftungsverbesserer und sonstige in solchen Mitteln an sich übliche Zusätze.

**Patentansprüche für den Vertragsstaat: AT**

1. Verfahren zur Herstellung von neuen Verbindungen der allgemeinen Formel

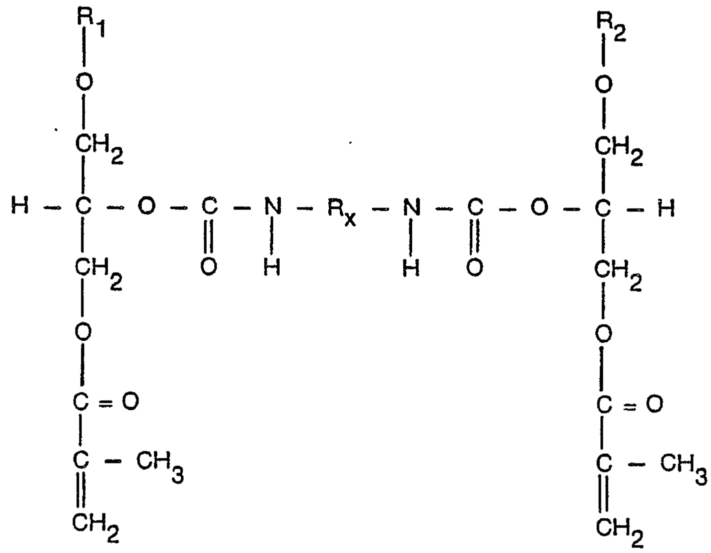

wobei $R_1$ und $R_2$ gleich oder verschieden sein können und eine geradkettige oder verzweigte Alkylgruppe mit 1 bis 14 C-Atomen, eine gegebenenfalls substituierte phenyl- oder Cycloalkylgruppe, und $R_x$ einen zweiwertigen aliphatischen, cycloaliphatischen, araliphatischen oder aromatischen Rest mit 2 bis 14 C-Atomen bedeuten, dadurch gekennzeichnet, daß man Diisocyanate der allgemeinen Formel

$$O{=}C{-}N{-}R_x{-}N{-}C{=}O$$

mit Methacroylalkylethern der allgemeinen Formel

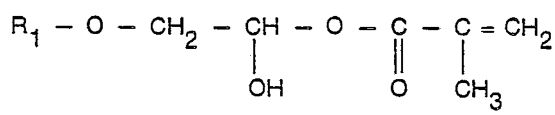

und / oder

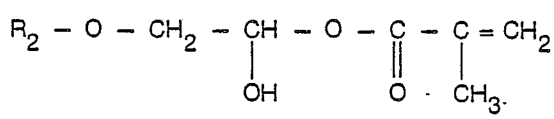

wobei $R_1$, $R_2$ und $R_x$ die oben angegebene Bedeutung haben, umsetzt.

2. Verfahren nach Anspruch 1, dadurch gekennzeichnet, daß $R_1$ und $R_2$ $C_2$—$C_8$-Alkylreste darstellen und $R_x$ einen Hexamethylenrest bedeutet.

3. Verfahren nach Anspruch 1, dadurch gekennzeichnet, daß $R_1$ und $R_2$ $C_2$—$C_8$-Alkylreste darstellen und $R_x$ einen Phenylen- oder Toluylenrest bedeutet.

4. Verfahren nach Anspruch 1, dadurch gekennzeichnet, daß $R_1$ und $R_2$ $C_2$—$C_8$-Alkylreste darstellen und $R_x$ eine Cycloalkylen- oder Methylenbiscycloalkylrest bedeutet.

5. Verfahren nach Anspruch 1, dadurch gekennzeichnet, daß $R_1$ und $R_2$ 2-Äthylhexylrest darstellen.

6. Verfahren nach Anspruch 1, dadurch gekennzeichnet, daß $R_1$ und $R_2$ gegebenenfalls substituierte Phenylreste darstellen und $R_x$ einen Hexamethylenrest bedeutet.

7. Verfahren nach Anspruch 1, dadurch gekennzeichnet, daß $R_1$ und $R_2$ gegebenenfalls substituierte Phenylreste und $R_x$ eine Phenylen- oder Toluylenrest bedeuten.

8. Verfahren nach Anspruch 1, dadurch gekennzeichnet, daß $R_1$ und $R_2$ gegebenenfalls substituierte Phenylreste und $R_x$ einen Cycloalkylen- oder Methylenbiscycloalkylrest bedeuten.

9. Verfahren nach Anspruch 1, dadurch gekennzeichnet, daß $R_1$ und $R_2$ gegebenenfalls substituierte Cycloalkyl-, insbesondere Cyclohexylreste, und $R_x$ einen Hexamethylenrest bedeuten.

10. Verfahren nach Anspruch 1, dadurch gekennzeichnet, daß $R_1$ und $R_2$ gegebenenfalls substituierte Cycloalkyl-, insbesondere Cyclohexylreste, und $R_x$ einen Phenylen- oder Toluylenrest bedeuten.

11. Verfahren nach Anspruch 1, dadurch gekennzeichnet, daß $R_1$ und $R_2$ gegebenenfalls substituierte Cycloalkyl-, insbesondere Cyclohexylreste, und $R_x$ einen Cycloalkylen- oder Methylen-biscycloalkylrest bedeuten.

12. Verwendung einer oder mehrerer nach den Ansprüchen 1 bis 11 hergestellter Verbindungen als Bindemittel.

13. Verwendung einer oder mehrerer nach den Ansprüchen 1 bis 11 hergestellter Verbindungen als Bindemittel in dentalen Behandlungsmaterialien.

14. Dentales Restorations- und Füllungsmaterial, dadurch gekennzeichnet, daß es neben Füllstoffen, Initiatoren, Beschleunigern und weiteren üblichen Bestandteilen mindestens eine nach den Ansprüchen 1 bis 11 hergestellte Verbindung enthält.

15. Dentales Restorations- und Füllungsmaterial nach Anspruch 14, dadurch gekennzeichnet, daß es folgende Bestandteile enthält:

9—90 Gew.-% mindestens einer Verbindung nach den Ansprüchen 1 bis 11;
0,01—2,5 Gew.-% mindestens eines Polymerisationsinitiators oder Polymerisationsbeschleunigers;
10—85 Gew.-% mindestens eines Füllstoffs;

sowie gegebenenfalls weitere polymerisierbare Verbindungen, UV-Absorber, Farbstoffe, Haftungsverbesserer und sonstige in solchen Mitteln an sich übliche Zusätze.

**Revendications pour les Etats contractantts: BE CH DE FR GB IT LI LU NL SE**

1. Composés de la formule générale

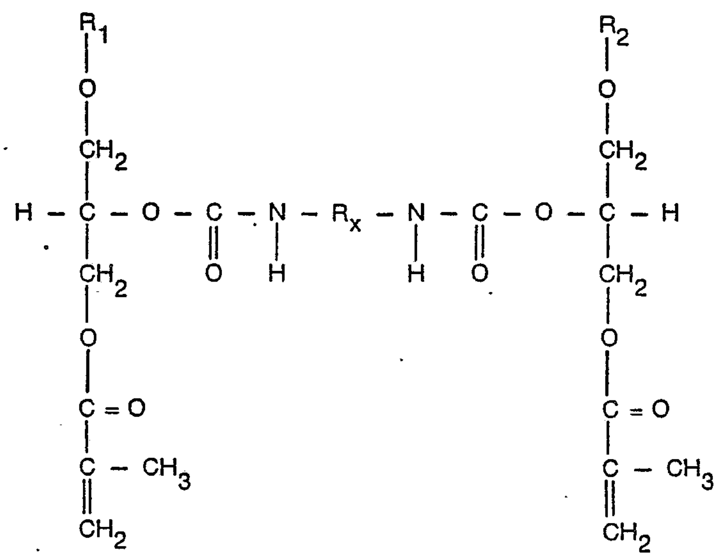

à quoi $R_1$ et $R_2$ sont identiques ou différents et représentent un groupe alkyle de 1 à 14 d'atomes de carbone en ligne droite ou ramifiée, un groupe phényle ou cycloalkyle, le cas échéant substitué, ou un groupe benzyle, et $R_x$ représente un groupe bivalent aliphatique, cycloaliphatique, araliphatique ou aromatique avec 2 à 14 atomes de carbone.

2. Composés de la formule générale selon revendication 1, à quoi $R_1$ et $R_2$ représentent des groupes alkyles avec 2 à 8 atomes de carbone et $R_x$ représente un groupe hexaméthylène.

3. Composés de la formule générale selon revendication 1, à quoi $R_1$ et $R_2$ représentent des groupes alkyles$^+$ et $R_x$ représente un groupe phénylène ou toluylène.

4. Composés de la formule générale selon revendication 1, à quoi $R_1$ et $R_2$ représentent des groupes alkyles$^+$ et $R_x$ représente un groupe cycloalkylène ou méthylènebiscycloalkyle.

5. Composés de la formule générale selon revendications 2 à 4, à quoi $R_1$ et $R_2$ représentent des groupes 2-éthylhexyles.

6. Composés de la formule générale selon revendication 1, à quoi $R_1$ et $R_2$ représentent des groupes phényles, substitués les cas écheant, et $R_x$ représente un groupe hexamèthylène.

7. Composés de la formule générale selon revendication 1, à quoi $R_1$ et $R_2$ représentent des groupes phényles, substitués le cas écheant, et $R_x$ représente un groupe phenylène ou toluylène.

---

$^+$ avec 2 à 8 atomes de carbone

8. Composés de la formule générale selon revendication 1, à quoi $R_1$ et $R_2$ représentent des groupes phényles, substitués le cas échéant, et $R_x$ représente un groupe cycloalkylène ou méthylènebiscycloalkyle.

9. Composés de la formule générale selon revendication 1, à quoi $R_1$ et $R_2$ représentent des groupes cycloalkyles, substitués le cas écheant, particulièrement des groupes cyclohexyliques, et $R_x$ représente un groupe hexaméthylène.

10. Composés de la formule générale selon revendication 1, à quoi $R_1$ et $R_2$ représentent des groupes cycloalkyles, substitués le cas écheant, particulièrement des groupes cyclohexyliques, et $R_x$ représente un groupe phenylène ou toluylène.

11. Composés de la formule générale selon revendication 1, à quoi $R_1$ et $R_2$ représentent des groupes cycloalkyles, substitués le cas écheant, particulièrement des groupes cyclohexyliques, et $R_x$ représente un groupe cycloalkylène ou méthylènebiscycloalkyle.

12. Usage d'un ou plusieurs composés selon les revendications 1 à 11 comme des liants.

13. Usage d'un ou plusieurs composés selon les revendications 1 à 11 comme des liants pour les matières dentaires.

14. Matière dentaire d'obturation contenant des charges, initiateurs, accélérateurs et ingrédients additionels usuels, caracterisée en ce contient au moins un composé selon les revendications 1 à 11.

15. Matière dentaire d'obturation selon revendication 14, caracterisée en ce contient les ingrédients suivants:

9—90% en poids au moins d'un composé selon les revendications 1 à 11;
0,01—2,5% en poids au moins d'un initiateur ou accélérateur de polymérisation;
10—85% en poids au moins d'une charge;

ainsi que, le cas échéant, des composés polymérisables additionels, des colorants, des agents améliorant d'adhésion, et des autres ingrédients usuels dans ces matières.

**Revendications pour l'Etat contractant: AT**

1. Procédé pour la préparation des composés nouveaux de la formule générale

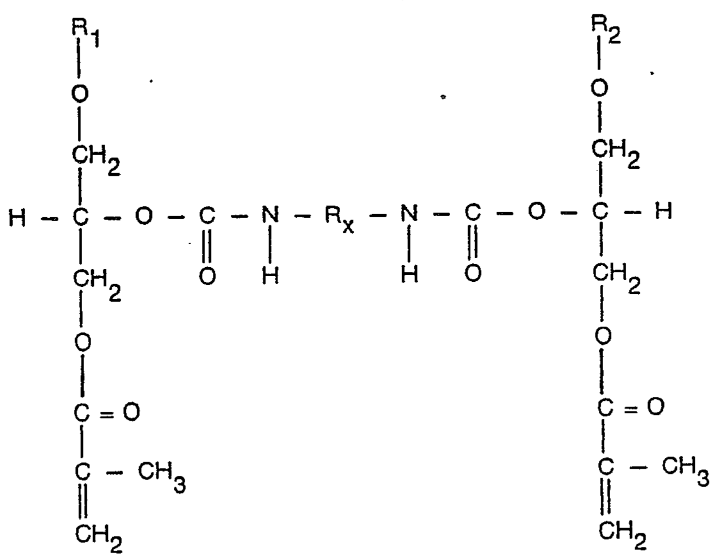

à quoi $R_1$ et $R_2$ sont identiques ou différents et représentent un groupe alkyle de 1 à 14 d'atomes de carbone en ligne droite ou ramifiée, un groupe phényle ou cycloalkyle, le cas échéant substitué, et $R_x$ représente un groupe bivalent aliphatique, cycloaliphatique, araliphatique ou aromatique avec 2 à 14 atomes de carbone, caracterisé en ce qu'on fait réagir des diisocyanates de la formule générale

$$O{=}C{-}N{-}R_x{-}N{-}C{=}O$$

avec des éthers methacroyliques de la formule générale

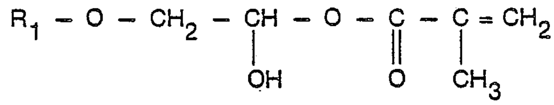

et / ou

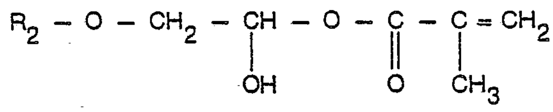

à quoi $R_1$, $R_2$ et $R_x$ ont la signification susmentionée.

2. Procédé selon revendication 1, à quoi $R_1$ et $R_2$ représentent des groupes alkyles avec 2 à 8 atomes de carbone et $R_x$ représente un groupe hexaméthylène.

3. Procédé selon revendication 1, à quoi $R_1$ et $R_2$ représentent des groupes alkyles avec 2 à 8 atomes de carbone et $R_x$ représente un groupe phénylène ou toluylène.

4. Procédé selon revendication 1, à quoi $R_1$ et $R_2$ représentent des groupes alkyles avec 2 à 8 atomes de carbone et $R_x$ représente un groupe cycloalkylène ou méthylènebiscycloalkyle.

5. Procédé selon revendication 1, à quoi $R_1$ et $R_2$ représentent des groupes 2-éthylhexyliques.

6. Procédé selon revendication 1, à quoi $R_1$ et $R_2$ représentent des groupes phényles, substitués le cas échéant, et $R_x$ représente un groupe hexaméthylène.

7. Procédé selon revendication 1, à quoi $R_1$ et $R_2$ représentent des groupes phényles, substitués le cas échéant, et $R_x$ représente un groupe phenylène ou toluylène.

8. Procédé selon revendication 1, à quoi $R_1$ et $R_2$ représentent des groupes phenyles, substitués le cas échéant, et $R_x$ représente un groupe cycloalkylène ou méthylènebiscycloalkyle.

9. Procédé selon revendication 1, à quoi $R_1$ et $R_2$ représentent des groupes cycloalkyles, substitués le cas échéant, particulièrement des groupes cyclohexyliques, et $R_x$ représente un groupe hexaméthylène.

10. Procédé selon revendication 1, à quoi $R_1$ et $R_2$ représentent des groupes cycloalkyles, le cas échéant substitués, particulièrement des groupes cyclohexyliques, et $R_x$ représente un groupe phenylène ou toluylène.

11. Procédé selon revendication 1, à quoi $R_1$ et $R_2$ représentent des groupes cycloalkyles, le cas échéant substitués, particulièrement des groupes cyclohexyliques, et $R_x$ représente un groupe cycloalkylène ou méthylènebiscycloalkyle.

12. Usage d'un ou plusieurs composés préparés selon les revendications 1 à 11 comme des liants.

13. Usage d'un ou plusieurs composés préparés selon les revendications 1 à 11 comme des liants pour les matières dentaires.

14. Matière dentaire d'obturation contenant des charges initiateurs, accélérateurs et ingrédients additionels usuels, caractérisée en ce contient au moins un composé préparé selon les revendications 1 à 11.

15. Matière dentaire d'obturation selon revendication 14, caractérisée en ce contient les ingrédients suivants:

9—90% en poids au moins d'un composé préparé selon les revendications 1 à 11;
0,01—2,5% en poids au moins d'un initiateur ou accélérateur de polymérisation;
10—85% en poids au moins d'une charge;

ainsi que, le cas échéant, composés polymérisables additionels, des colorants, des agents améliorant d'adhésion, et des autres ingrédients usuels dans ces matières.

**Claims for the Contracting States: BE CH DE FR GB IT LI LU NL SE**

1. Compounds of the general formula

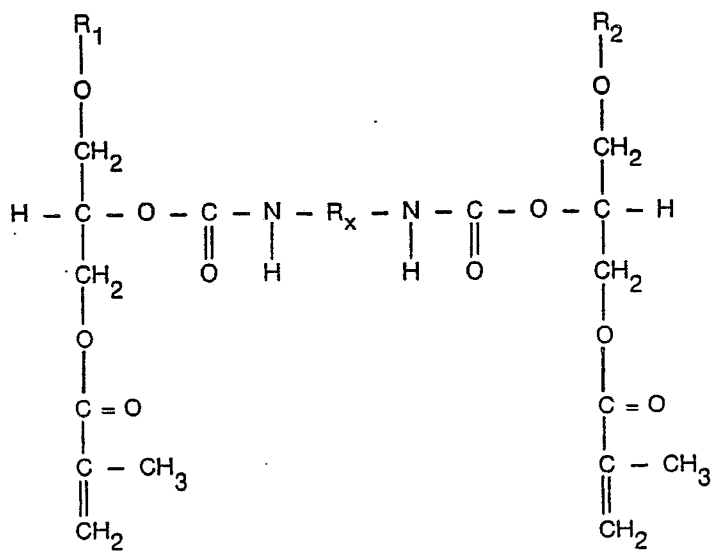

where $R_1$ and $R_2$ may be identical or different and are representing a straight or branched alkyl group from 1 to 14 C-atoms, an optionally substituted phenyl or cycloalkyl group, a benzyl group, and $R_x$ is representing a divalent aliphatic, cycloaliphatic, araliphatic or aromatic group with 2 to 14 C-atoms.

2. Compounds of the general formula of claim 1, where $R_1$ and $R_2$ are representing $C_2$- to $C_8$-alkyl groups and $R_x$ is representing a hexamethylene group.

3. Compounds of the general formula of claim 1, where $R_1$ and $R_2$ are representing $C_2$- to $C_8$-alkyl groups and $R_x$ is representing a phenylene or toluylene group.

4. Compounds of the general formula of claim 1, where $R_1$ and $R_2$ are representing $C_2$—$C_8$-alkyl groups and $R_x$ is representing a cycloalkylene or methylenebiscycloalkyl group.

5. Compounds of the general formula according to claims 2 to 4, where $R_1$ and $R_2$ are representing 2-ethylhexyl groups.

6. Compounds of the general formula of claim 1, where $R_1$ and $R_2$ are representing optionally substituted phenyl groups and $R_x$ is representing a hexamethylene group.

7. Compounds of the general formula of claim 1, where $R_1$ and $R_2$ are representing optionally substituted phenyl groups and $R_x$ is representing a phenylene or toluylene group.

8. Compounds of the general formula of claim 1, where $R_1$ and $R_2$ are representing optionally substituted phenyl groups and $R_x$ is representing a cycloalkylene or methylenebiscycloalkyl group.

9. Compounds of the general formula of claim 1, where $R_1$ and $R_2$ are representing an optionally substituted cycloalkyl group, especially a cyclohexyl group, and $R_x$ is representing a hexamethylene group.

10. Compounds of the general formula of claim 1, where $R_1$ and $R_2$ are representing an optionally substituted cycloalkyl group, especially a cyclohexyl group, and $R_x$ is representing a phenylene or toluylene group.

11. Compounds of the general formula of claim 1, where $R_1$ and $R_2$ are representing an optionally substituted cycloalkyl group, especially a cyclohexyl group, and $R_x$ is representing a cycloalkylene or methylenebiscycloalkyl group.

12. Use of one or more compounds according to the claims 1 to 11 as binding agents.

13. Use of one or more compounds according to claims 1 to 11 as binding agents in dental materials.

14. Dental restoring and filling material, containing in addition to fillers, initiators, accelerators, and common components at least one component according to claims 1 to 11.

15. Dental restoring and filling material according to claim 14, containing the following components:

9—90% by weight of at least one compound according to claims 1 to 11;
0,01—2,5% by weight of at least one polymerization initiator or polymerization accelerator;
10—85% by weight of at least one filler;

optionally additional polymerizable compounds, UV-absorbers, dyes, adhesion improvers and additional compounds being useful in such compositions.

**Claims for the Contracting State: AT**

1. Process for the preparation of new compounds of the general formula

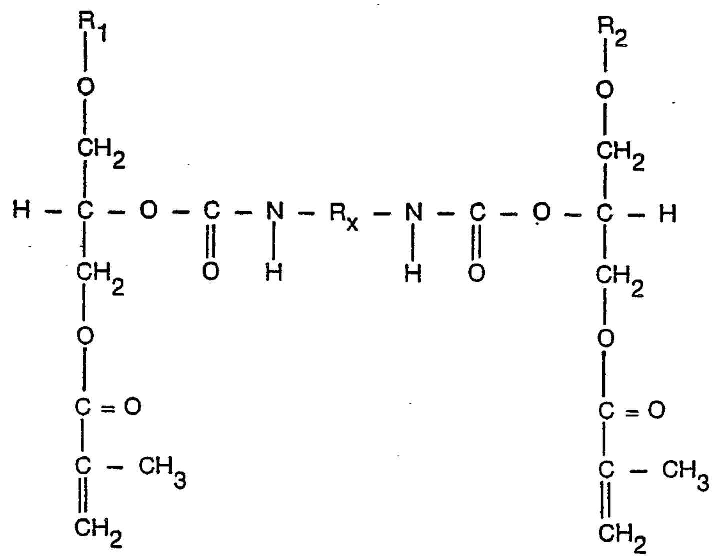

where $R_1$ and $R_2$ may be identical or different and are representing a straight or branched alkyl group from 1 to 14 C-atoms, an optionally substituted phenyl or cycloalkyl group, and $R_x$ is representing a divalent aliphatic, cycloaliphatic, araliphatic or aromatic group with 2 to 14 C-atoms, characterized in that diisocyanates of the general formula

$$O{=}C{-}N{-}R_x{-}N{-}C{=}O$$

are reacted with methacroyl alkyl ethers of the general formula

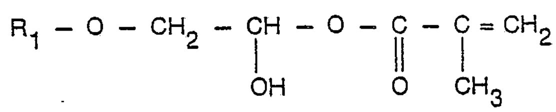

and /or

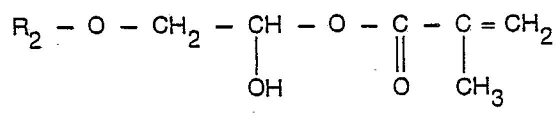

where $R_1$, $R_2$ and $R_x$ have the same meaning as identified above.

2. Process according to claim 1, where $R_1$ and $R_2$ are representing $C_2$- to $C_8$-alkyl groups and $R_x$ is representing a hexamethylene group.

3. Process according to claim 1, where $R_1$ and $R_2$ are representing $C_2$- to $C_8$-alkyl groups and $R_x$ is representing a phenylene or toluylene group.

4. Process according to claim 1, where $R_1$ and $R_2$ are representing $C_2$—$C_8$-alkyl groups and $R_x$ is representing a cycloalkylene or methylenebiscycloalkyl group.

5. Process according to claim 1, where $R_1$ and $R_2$ are representing 2-ethylhexyl groups.

6. Process according to claim 1, where $R_1$ and $R_2$ are representing optionally substituted phenyl groups and $R_x$ is representing a hexamethylene group.

7. Process according to claim 1, where $R_1$ and $R_2$ are representing optionally substituted phenyl groups and $R_x$ is representing a phenylene or toluylene group.

8. Process according to claim 1, where $R_1$ and $R_2$ are representing optionally substituted phenyl groups and $R_x$ is representing a cycloalkylene or methylenebiscycloalkyl group.

9. Process according to claim 1, where $R_1$ and $R_2$ are representing an optionally substituted cycloalkyl group, especially a cyclohexyl group, and $R_x$ is representing a hexamethylene group.

10. Process according to claim 1, where $R_1$ and $R_2$ are representing an optionally substituted cycloalkyl group, especially a cyclohexyl group, and $R_x$ is representing a phenylene or toluylene group.

11. Process according to claim 1, where $R_1$ and $R_2$ are representing an optionally substituted cycloalkyl group, especially a cyclohexyl group, and $R_x$ is representing a cycloalkylene or methylenebiscycloalkyl group.

12. Use of one or more compounds prepared according to claims 1 to 11 as binding agents.

13. Use of one or more compounds prepared according to claims 1 to 11 as binding agents in dental materials.

14. Dental restoring and filling material, containing in addition to fillers, initiators, accelerators, and common components at least one component prepared according to claims 1 to 11.

15. Dental restoring and filling material according to claim 14, containing the following components:

9—90% by weight of at least one compound prepared according to claims 1 to 11;
0,01—2,5% by weight of at least one polymerization initiator or polymerization accelerator;
10—85% by weight of at least one filler;

optionally additional polymerizable compounds, UV-absorbers, dyes, adhesion improvers and additional compounds being useful in such compositions.